# EUROPEAN PATENT APPLICATION

(11) **EP 3 415 506 A1**
(43) Date of publication of application: **19.12.2018**
(21) Application number: 17175758.6
(22) Date of filing: 13.06.2017
(51) Int. Cl.: C07D 285/12, C07D 285/125, C07D 285/13, C07C 337/02

(54) **PROCESS FOR THE MANUFACTURE OF HALOALKYL SUBSTITUTED THIADIAZOLE COMPOUNDS**

(71) Applicant: SOLVAY SA, 1120 Brussels (BE)
(72) Inventor: BRAUN, Max Josef, 30900 Wedemark (DE); JAUNZEMS, Janis, 30559 Hannover (DE); METZ, François, 69540 Irigny (FR)
(74) Representative: Mross, Stefan P.M.

(57) **Abstract**

The present invention concerns processes for the manufacture of haloalkyl substituted thiadiazole compounds and processes for the manufacture of agriculturally or pharmaceutically active compounds comprising the processes for the manufacture of haloalkyl substituted thiadiazole compounds.

## Description

The present invention concerns processes for the manufacture of haloalkyl substituted thiadiazole compounds and processes for the manufacture of agriculturally or pharmaceutically active compounds comprising the processes for the manufacture of haloalkyl substituted thiadiazole compounds.

Halogenalkyl substituted thiadiazole moieties have been recognized to be useful structural moieties in numerous agriculturally or pharmaceutically active compounds, such as, for example Flufenacet, a herbicide. The routes towards compounds bearing the halogenalkyl substituted thiadiazole moieties have so far not been available starting from the readily available, good to handle haloalkylcarboxylic acid halides. EP0926141, for example, introduces the CF₃ group with trifluoroacetic acid. The newly developed process toward of haloalkyl substituted thiadiazole compounds starting from haloalkylcarboxylic acid halides have advantageous properties, such as short routes, good to handle starting materials, commercially available starting materials such as the haloalkylcarboxylic acid halides, and overall good yields and waste optimization.

A first object of the present invention thus is a process for the manufacture of a compound of formula (I), which comprises a step wherein a compound of formula (II) is reacted with a compound of formula (III) 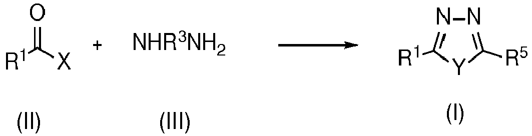
wherein R¹ is a halogenated C₁₋₄ alkyl group,
Y is -S- or -S(O₂)-, R³ is selected from the group consisting of H and - C(S)-R², wherein R² is the group -QR⁴ wherein R⁴ is a C₁₋₄ alkyl group,
   wherein when R³ is -C(S)-R², wherein Q is S or O, and R⁴ is a C₁₋₄ alkyl group, R⁵ in (I) is the group R²
   wherein when R³ is H, R⁵ in (I) is the group H
and wherein X is selected from the group consisting of F, Cl, Br and I.

Another object of the present invention is a process for the manufacture of a compound of formula (XIV)), which comprises a step wherein a compound of formula (II) is reacted with a compound of formula (III) 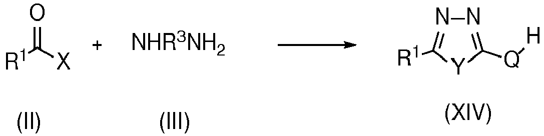 wherein R¹ is a halogenated C₁₋₄ alkyl group, Y is -S- or -S(O₂)-, wherein R³ is -C(S)-R², wherein R² is the group -QR⁴ wherein Q is S or O, and R⁴ is a C₁₋₄ alkyl group, and wherein a base is present in the reaction of (II) and (III).

Yet another object of the present invention are processes for the manufacture of a compound of formula (VI) 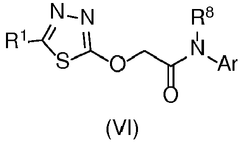 wherein R⁸ is selected from the group consisting of H, optionally substituted branched or straight C₁-C₁₂-alkyl, optionally substituted C₃₋₈ cycloalkyl, optionally substituted aryl, optionally substituted heterocycloalkyl, and optionally substituted heteroaryl, and wherein Ar is an optionally substituted aryl or heteroaryl group, which comprises the processes for the manufacture of a compound of formula (I) or (XIV) and optionally further steps.

The invention further concerns a process for the manufacturing of an agriculturally or pharmaceutically active compound or a composition comprising an agriculturally or pharmaceutically active compound, which comprises any of the processes for the manufacture of a compound of formula (I), (VI) or (XIV), in particular wherein the agriculturally active compound is N-(4-Fluorophenyl)-N-isopropyl-2-{[5-(trifluoromethyl)-1,3,4-thiadiazol-2-yl]oxy}acetamide or a precursor thereof.

In the present invention, designations in singular are in intended to include the plural; for example, "a solvent" is intended to denote also "more than one solvent" or "a plurality of solvents".

All aspects and embodiments of the present invention are combinable.

In the context of the present invention, the term "comprising" is intended to include the meaning of "consisting of'.

When a double bond is depicted in a particular E/Z geometry, this is intended to also denote the other geometric form as well as mixtures thereof. Generally, in the present invention, basic compounds, such as the compound of formula (III) or (XIII), can also be used in their form as a salt, for example in the form of a hydrogen halide salt.

In the context of the present invention, the term "C₁-C₁₂-alkyl" is intended to denote straight or branched alkyl groups having one to twelve carbon atoms. The group comprises, for example, methyl, ethyl, n-propyl, isopropyl, n-, iso-, sec- and t-butyl, n-pentyl and its isomers, n-hexyl and its isomers, 1,3-dimethylbutyl, 3,3-dimethylbutyl, n-heptyl and its isomers, n-octyl and its isomers, n-nonyl and its isomers, n-decyl and its isomers, n-undecyl and its isomers and n-dodecyl and its isomers. Often, the C₁ to C₄ alkyl groups are the most preferred groups of the C₁-C₁₂ alkyl group. The term "C₁-C₄-alkyl" is intended to denote straight or branched alkyl groups having one to four carbon atoms. This group comprises methyl, ethyl, n-propyl, isopropyl, n-, iso-, sec- and t-butyl.

The term "C₃-C₈ cycloalkyl group" intends to denote mono-, bi- or tricyclic hydrocarbon groups comprising 3 to 10 carbon atoms, especially 3 to 6 carbon atoms. Examples of monocyclic groups include cyclopropyl, cyclobutyl, cyclopentyl, cyclohexyl, cycloheptyl or cyclooctyl. Examples of bicyclic groups include bicyclo[2.2.1]heptyl, bicyclo[3.1.1]heptyl, bicyclo[2.2.2]octyl and bicyclo[3.2.1]octyl. Examples of tricyclic groups are adamantyl and homoadamantyl.

The term "aryl group" intends to denote C₅-C₁₈ monocyclic and polycyclic aromatic hydrocarbons with 5 to 18 carbon atoms in the cyclic system. Specifically, this definition comprises, for example, the meanings cyclopentadienyl, phenyl, cycloheptatrienyl, cyclooctatetraenyl, naphthyl and anthracenyl.

In the context of the present invention, the term "heteroaryl" intends to denote C₅-C₁₈ monocyclic and polycyclic aromatic hydrocarbons with 5 to 18 carbon atoms in the cyclic system, wherein one or more methine (-C=) and/or vinylene (-CH=CH-) groups are replaced by trivalent or divalent heteroatoms, in particular nitrogen, oxygen and/or sulphur, respectively, in such a way as to maintain the continuous π-electron system characteristic of aromatic systems. Specifically, this definition comprises, for example, the meanings 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl; 1-pyrrolyl, 1-pyrazolyl, 1,2,4-triazol-1-yl, 1-imidazolyl, 1,2,3-triazol-l-yl, 1,3,4-triazol-1-yl; 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

A C₁-C₄ alkyl group, C₁-C₁₂ alkyl group, C₃-C₈ cycloalkyl group, aryl or heteroaryl group each can be optionally substituted by one or more substituents of the following group consisting of R', -X", -OR', -SR', -NR'₂, -SiR'₃, -COOR', - (C-O)R', -CN and -CONR'₂, wherein R' is selected independently, from the group consisting of hydrogen or C₁-C₁₂-alkyl group and X" is selected from the group consisting of F, Cl, Br, or I.

According to the present invention, if a reaction of two or more compounds to obtain a new compound is described, such as the reaction of compound (II) with (III) to obtain a compound of formula (I), it is understood that these reactions can proceed via one or more intermediary compounds and/or one or more intermediary steps.

One object of the present invention is a process for the manufacture of a compound of formula (I), which comprises a step wherein a compound of formula (II) is reacted with a compound of formula (III) 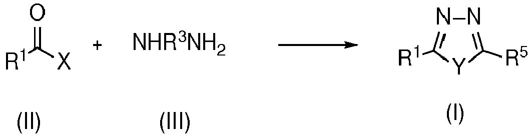
wherein R¹ is a halogenated C₁₋₄ alkyl group,
Y is -S- or -S(O₂)-, R³ is selected from the group consisting of H and - C(S)-R², wherein R² is the group -QR⁴ wherein R⁴ is a C₁₋₄ alkyl group,
   wherein when R³ is -C(S)-R², wherein Q is S or O, and R⁴ is a C₁₋₄ alkyl group, R⁵ in (I) is the group R²
   wherein when R³ is H, R⁵ in (I) is the group H
and wherein X is selected from the group consisting of F, Cl, Br and I.

The term "halogenated C₁₋₄ alkyl group" intends to denote that the C₁₋₄ alkyl group is substituted with at least one halogen atom, preferably with two or more halogen atoms, and more preferably with at least three halogen atom. In a preferred aspect, R¹ is a fluorinated C₁₋₄ alkyl group. In a more preferred aspect, R¹ is selected from the group consisting of CF₃, CF₂H, CF₂Cl, CFCl₂ and CCl₃, wherein CF₃ is most preferred.

R⁴ preferably is methyl, ethyl, n-propyl or isopropyl, wherein methyl and ethyl are preferred.

X preferably is Cl or F, and most preferably Cl.

Another object of the present invention is a process for the manufacture of a compound of formula (XIV)), which comprises a step wherein a compound of formula (II) is reacted with a compound of formula (III) 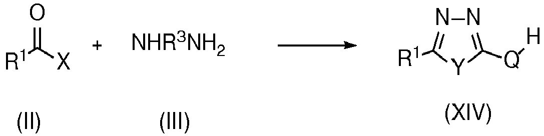 wherein R¹ is a halogenated C₁₋₄ alkyl group, Y is -S- or -S(O₂)-, wherein R³ is -C(S)-R², wherein R² is the group -QR⁴ wherein Q is S or O, and R⁴ is a C₁₋₄ alkyl group, and wherein a base is present in the reaction of (II) and (III). Due to the presence of a base, the group -QR⁴ is cleaved to yield the group -QH. « A base » intends to denote a base other than the compound of formula (III), which chemically also could be considered to be a base. « A base » can be, for example, an organic base, such as for example triethylamine or pyridine, or an inorganic base, such as for example KOH, NaOH, a metal alcoholate such as tBuOK and LiOH. The preferred groups R¹, R³, R², R⁴ and X are the same as described above.

In one aspect of the present invention, the invention concerns a process for the manufacture of a compound of formula (I) as described above, wherein R³ in (III) is H, the compound of formula (IV) obtained by the reaction of (II) and (III) is reacted with CH(OR⁶)₃, wherein R⁶ is an optionally substituted C₁₋₄ alkyl group, 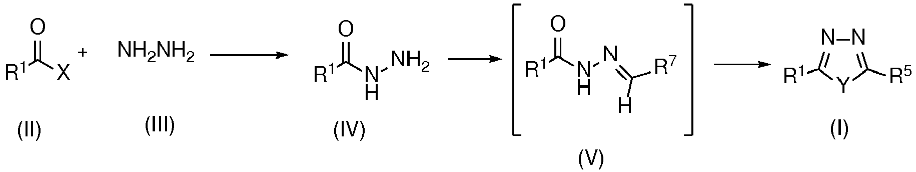 with a subsequent reaction with a compound selected from the group consisting of SCl₂, S₂Cl₂, SO₂Cl₂ or SO₂ClF to obtain the compound of formula (V), wherein R⁷ in the compound of formula (V) is -SCl or -S(O)₂Cl, which is cyclized, optionally in the presence of a base, into compound (I) wherein R⁵ is H and R¹ and Y are as defined above. R⁶ preferably is methyl or ethyl, wherein ethyl is most preferred. The reaction of (II) and (III) is optionally performed in the presence of a base. The compound of formula (III), wherein R³ is H, can be used neat or in a solvent, wherein water is the preferred solvent for (III).

Another object of the present invention is a process for the manufacture of a compound of formula (VI), 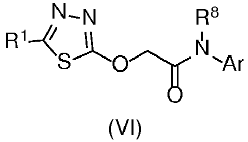 which comprises the process of the manufacture of compound (I) from (II) and (III), wherein R³ is H, wherein the compound of formula (IV) is reacted with CH(OR⁶)₃, wherein R⁶ is an optionally substituted C₁₋₄ alkyl group, with a subsequent reaction with a compound SCl₂ or S₂Cl₂ to obtain the compound of formula (V) wherein R⁷ is -SCl, which is cyclized, optionally in the presence of a base, into compound (I) wherein R⁵ is H, R¹ is as defined in claim 1 and Y is - S-, and which further comprises a step of halogenating the compound of formula (I) as obtained in the reaction of a compound of formula (II) and (III) with R³ = H as described above, with a halogenating agent, wherein a suitable halogenating agent is selected from Cl₂, F₂, Br₂, HCl, HBr, HF or any other suitable halogenating agent, 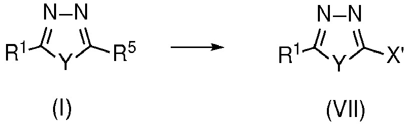 wherein X' is selected from F, Cl and Br, wherein Br is preferred, and which comprises a further step in which a compound of formula (VIII) is reacted with a compound of formula (VII), optionally in the presence of a base, to obtain a compound of formula (VI) 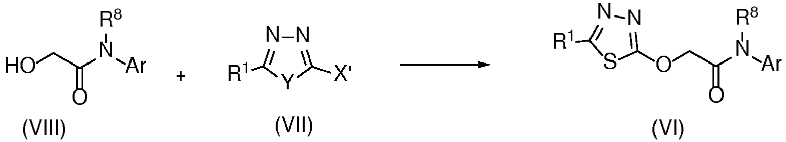 wherein R⁸ is selected from the group consisting of H, optionally substituted branched or straight C₁-C₁₂-alkyl, optionally substituted C₃₋₈ cycloalkyl, optionally substituted aryl, optionally substituted heterocycloalkyl, and optionally substituted heteroaryl, and wherein Ar is an optionally substituted aryl or heteroaryl group. R⁸ preferably is an optionally substituted C₁₋₄ alkyl group, wherein isopropyl is most preferred. Ar preferably is a substituted aryl group, more preferably an optionally substituted phenyl group, even more preferably a halogenated phenyl group, and most preferably a 4-Fluorophenyl group. R⁶ preferably is a methyl or ethyl group.

Another object of the present invention is a process for the manufacture of a compound of formula (VI), 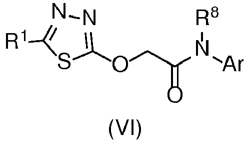 which comprises the process of the manufacture of compound (I) from (II) and (III), wherein the compound of formula (IV) is reacted with CH(OR⁶)₃, wherein R⁶ is defined as above, with a subsequent reaction with a compound - SO₂Cl₂ or -SO₂ClF to obtain the compound of formula (V) wherein R⁷ is -SO₂Cl, which is cyclized, optionally in the presence of a base, into compound (I) wherein R⁵ is H, R¹ is as defined as above and Y is -S(O)₂-, which further comprises a step wherein the compound of formula (I), wherein Y is -S(O)₂- and R⁵ is H, is reduced with a suitable reducing agent to a group of Y which is -S-, wherein the suitable reducing agent can be selected, for example, from NaBH₄, LiAlH₄, NaH, diisobutylaluminium hydride, silyl hydrides, phosphorous hydrides such as phosphine, Raney nickel and H₂ and Raney nickel in formic acid, wherein NaBH₄ and LiAlH₄ are preferred; 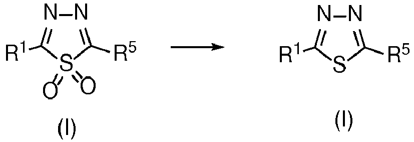 which further comprises a step of halogenating the compound of formula (I) with a halogenating agent, wherein a suitable halogenating agent is selected, for example, from Cl₂, F₂, Br₂, HCl, HBr, HF or any other suitable halogenating agent, 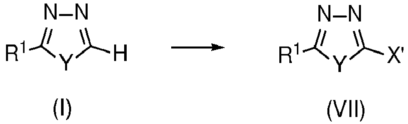 wherein X' is selected from F, Cl and Br
and which comprises a further step in which a compound of formula (VIII) is reacted with a compound of formula (VII), optionally in the presence of a base, to obtain a compound of formula (VI) 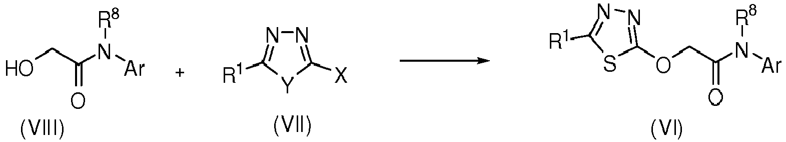 wherein R⁸, R¹ and Ar are defined as above.

Another object of the present invention is a process for the manufacture of formula (I), wherein in the compound of formula (III), R³ is -C(S)-R², R² is the group -QR⁴, wherein Q is S or O, wherein S is the preferred Q, and R⁴ is a C₁₋₄ alkyl group, and wherein in the reaction of compound (III) with compound (II), a compound of formula (IX) is obtained, 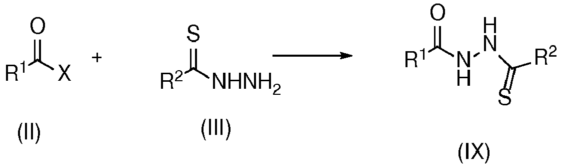 wherein the compound of formula (IX) then is cyclized, optionally in the presence of a base, to obtain a compound of formula (I) where Y is S and R¹ is defined as above 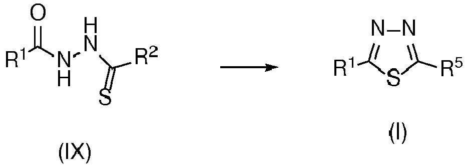 wherein R⁵ in compound (I) the group R², which is the group -QR⁴ wherein Q is S or O, and wherein R⁴ is a C₁₋₄ alkyl group. The groups of formula R¹ and preferred groups of formula R⁴ are defined as above. When a base is present in the cyclization of formula (IX), the base can be organic or inorganic. The base can be selected, for example, from triethylamine or pyridine, KOH, NaOH and LiOH. The manufacture of compound of formula (III), R³ is -C(S)-R², R² is the group -QR⁴, wherein Q is S or O, wherein the preferred Q is S, and R⁴ is a C₁₋₄ alkyl group, is described, for example, in EP841325.

In one aspect of the present invention, the invention concerns a process for the manufacture of a compound of formula (VI), 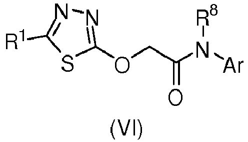 which comprises the process which comprises the reaction of compound (III) with compound (II), wherein a compound of formula (IX) is obtained, wherein the compound of formula (I) in which R¹ is defined as above, and R² is the group -QR⁴, wherein Q is S, and wherein R⁴ is a C₁₋₄ alkyl group, is reacted with and oxidant, for example for example 3-chloroperbenzoic acid (mCPBA), KMnO₄, oxygen or H₂O₂, optionally in the presence of to obtain a compound of formula (X) 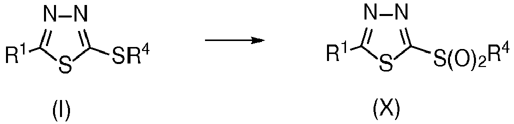 wherein R¹ is defined as above,
and wherein the compound of formula (X) is subsequently reacted with a base, such as, for example, NaH, t-BuOK, NaOH, KOH, LiOH or TDA (Tris[2-(2-methoxyethoxy)ethyl]amine), wherein TDA in sulfolane are preferred, followed by a reaction with a compound of formula (VIII) 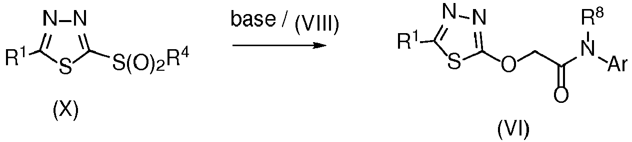 wherein the compound according to formula (VIII) and preferred residues R¹, R⁴, R⁸ and Ar are as defined as above.

Another object of the present invention is a process for the manufacture of a compound of formula (VI), 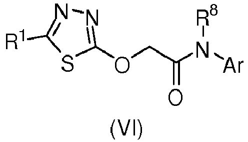 wherein R¹, R⁸ and Ar are defined as above, which comprises the process for the manufacture of a compound of formula (I) or (XIV), wherein R¹ R³, R⁵, Q and X are defined as above,
which further comprises a step wherein the compound of formula (I) R² is the group -QR⁴ wherein Q is S, and wherein R⁴ is a C₁₋₄ alkyl group, and R¹ is defined as above
or which comprises the step wherein a compound of formula (XIV) wherein Q is O, and R¹ is defined as above
is reacted with a compound of formula (XI), optionally in the presence of a base to obtain a compound of formula (XII) 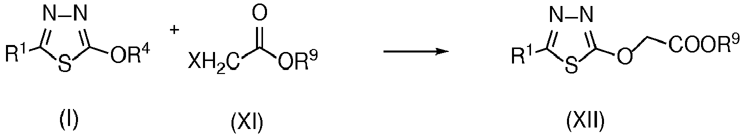 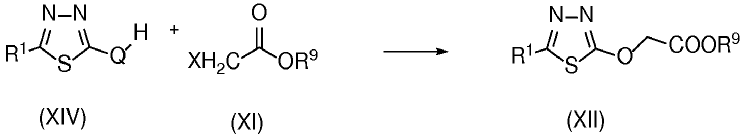 wherein X in compound (XI) is selected from F, Cl and Br, and wherein R⁹ is a C₁₋₁₂ alkyl group
wherein X in compound (XI) is selected from F, Cl and Br, and wherein R⁹ is an optionally substituted C₁₋₁₂ alkyl group,
and which further comprises a step of reacting the compound of formula (XII) with a compound of formula (XIII), optionally in the presence of a base, to obtain the compound of formula (VI), wherein Ar and R⁸ in (XIII) are defined as above.

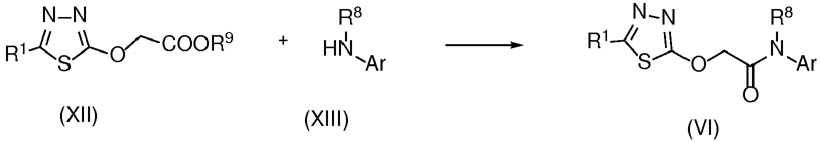

The base that is optionally present in the step of reacting (XII) and (XIII) generally is a base other than the compound of formula (XIII), which chemically also could be considered to be a base. « A base » can be, for example, an organic base, such as for example triethylamine or pyridine, or an inorganic base, such as for example KOH, NaOH or LiOH. Optionally, in the process as described above, the compound of formula (I) is obtained through a process wherein in the reaction of (II) and (III) in order to obtain (I), intermediary compound (IX) is obtained as described above.

Another object of the present invention is a process for the manufacture of a compound of formula (VI), wherein a compound of (XIII) is reacted with a compound of formula (XIII), wherein the compound of formula (XII) is converted, prior to the reaction with the compound of formula (XIII), into a compound of formula (XV), with subsequent activation by reaction with a halogenating agent or activation as anhydride to obtain a compound of formula (XV), before reaction with the compound of formula (XIII) to obtain the compound of formula (VI), wherein R¹⁰ is selected from the group consisting of X', wherein X' is selected from the group consisting of Cl, Br or F, and the group -O-C(O)-R¹¹, wherein R¹¹ is an optionally substituted C₁₋₄ alkyl group or an optionally substituted aryl group
or R¹⁰ is the group R^{10'} =

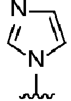

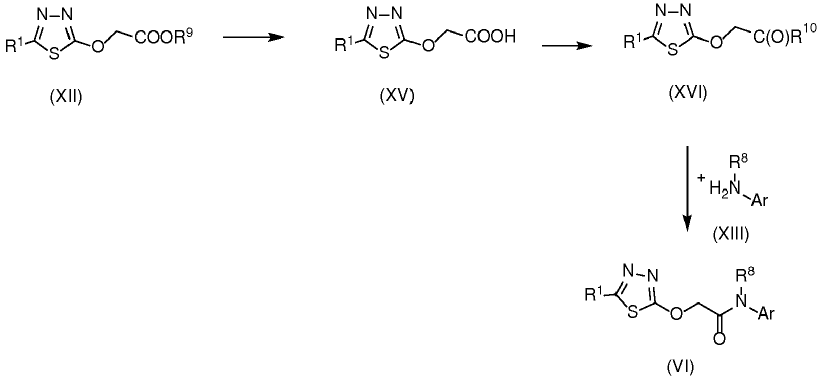

The reaction wherein the compound of formula (XII) is converted, prior to the reaction with the compound of formula (XIII), into a compound of formula (XV), can be effected, for example, by basic or acidic conversion of the ester group -COOR⁹ into the group -COOH, for example, by treatment with a base such as KOH, NaOH or LiOH, or by treatment with a suitable acid such as HCl or HBr. The conversion of compound (XV) into the compound of formula (XVI) wherein R¹⁰ is selected from X', in particular Cl, can be effected by suitable halogenation agents, such as oxalyl chloride, thionyl chloride or other agents which are suitable to convert a carboxylic group into a carboylic halide group. Optionally the conversion is effected in the presence of a base and/or a catalyst, such as DMF. When R¹⁰ is R^{10'}, the conversion of compound (XV) into the compound of formula (XVI) wherein R¹⁰ is R¹⁰ is effected by the reaction of (XV) with 1,1'-Carbonyldiimidazole (CDI). The reaction of (XVI) and (XIII) to obtain the compound of formula (VI) can optionally be effected in the presence of a base, wherein the base generally is a base other than the compound of formula (XIII), which chemically also could be considered to be a base. «A base » can be, for example, an organic base, such as for example triethylamine or pyridine, or an inorganic base, such as for example KOH, NaOH or LiOH.

In all of the processes according to the present invention which concern a process for the manufacture of a compound of formula (VI), R⁸ preferably is an optionally substituted C₁₋₄ alkyl group, preferably R⁸ is an isopropyl group.

The invention further concerns a process for the manufacturing of an agriculturally or pharmaceutically active compound or a composition comprising an agriculturally or pharmaceutically active compound, which comprises at least one of the processes according to the present invention for the manufacture of a compound of formula (I), (XIV), or (VI). In particular, the agriculturally active compound is N-(4-Fluorophenyl)-N-isopropyl-2-{ [5-(trifluoromethyl)-1,3,4-thiadiazol-2-yl]oxy}acetamide or a precursor thereof.

Should the disclosure of any patents, patent applications, and publications which are incorporated herein by reference conflict with the description of the present application to the extent that it may render a term unclear, the present description shall take precedence.

The following examples are intended to further explain the invention without limiting it.

The starting materials for the processes and steps according to the present invention can be obtained from commercial sources or can be manufactured according to procedures known from the public domain. The manufacture of compounds of formula (VIII) is described, for example, in EP0060426.

### Example 1 : 2,2,2-trifluoroacetohydrazide

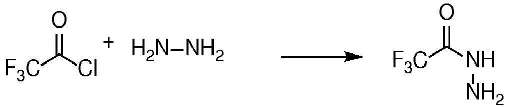

To a solution of hydrazine (48.3 mmol) and triethylamine (7. 26 g, 71. 8 mmol) in dichloromethane (10 mL) is added 2,2,2-trifluoroacetyl chloride (47.8 mmol) at 0°C. The mixture is stirred for 16 hours at ambient temperature, and the resulting suspension is filtered and washed with dichloromethane. The filtrate is concentrated under reduced pressure to provide 2,2,2-trifluoroacetohydrazide.

### Example 2 : 2-(trifluoromethyl)-1,3,4-thiadiazole 1,1-dioxide

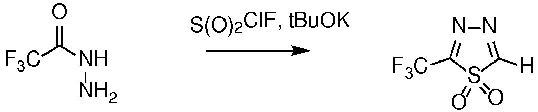

50 mmol of 2,2,2-trifluoroacetohydrazide are solved in dry THF, 1.2 eq triethylamine are added and the mixture is cooled to 0°C. 1.05 eq of sulfuryl chloride fluoride are slowly added at 0°C. The mixture is warmed to RT, stirred for 8 hours, 1.2 eq potassium tert-butylate 1 M in THF is added, and the mixture is stirred for 12 hours at 50°C. The reaction mixture is cooled to RT, washed with IN aq. HCl, aq. NaHCO₃ and water, dried over Na₂SO₄, and the solvents are evaporated to yield the crude product.

### Example 3: 2-(trifluoromethyl)-1,3,4-thiadiazole

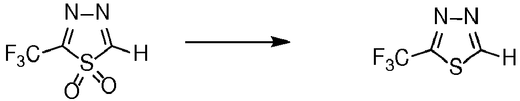

0.20 g (5.27 mmol) LiAlH₄ are added carefully to a solution of 2-(trifluoromethyl)-1,3,4-thiadiazole 1,1-dioxide (1.34 mmol) in diethyl ether (20 mL). The solution is refluxed for 30 min, cooled to room temperature and water is added, carefully, followed by 3 mL of conc. HCl. The phases are separated, organic phase washed with water and brine, dried over Na₂SO₄ and the diethyl ether is removed under reduced pressure to obtain the crude product.

### Example 4 :2-bromo-5-(trifluoromethyl)-1,3,4-thiadiazole

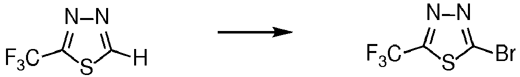

To a solution of 2-(trifluoromethyl)-1,3,4-thiadiazole (1.0 mol) in CH₃COOH (400 mL) is slowly added bromine (51 mL, 1.0 mol) at room temperature. The mixture is allowed to react for 5 hrs at 60°C, followed by removal of solvent under reduced pressure. The concentrated residue is neutralized with an aqueous solution of sodium hydrogencarbonate, extracted with dichloromethane DCM, dried and evaporated to afford the crude product.

### Example 5 :N-(4-fluorophenyl)-N-isopropyl-2-((5-(trifluoromethyl)-1,3,4-thiadiazol-2-yl)oxy)acetamide

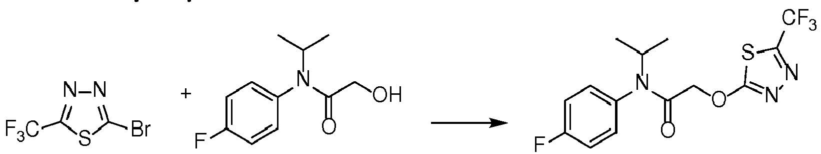

0.07 mol of N-(4-fluorophenyl)-2-hydroxy-N-isopropylacetamide and 4.5 g KOH are dispersed in 100 mL CH₃CN, cooled to 0°C and treated at 10°C with a solution of 2-bromo-5-(trifluoromethyl)-1,3,4-thiadiazole (1.1 eq) in 50 mL CH₃CN. The mixture is stirred at 30-40°C for 15 hours, the solvents evaporated, the residue is taken up in DCM, washed with IN HCl, aq. NaHCO₃, water, dried over MgSO₄ and the DCM evaporated to yield the crude product.

### Example 6 :N-(2,2,2-trifluoroacetyl)formohydrazonic hypochlorous thioanhydride

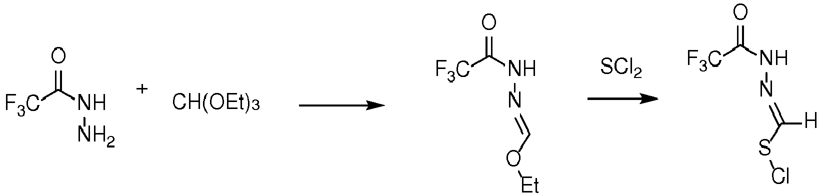

A solution of 2,2,2-trifluoroacetohydrazide (19,2 mmol mol) in triethyl orthoformate (100 mL) is stirred at 110°C for 12 hours. The solution is cooled to room temperature and then concentrated under reduced pressure to give the crude ethyl *N*-(2,2,2-trifluoroacetyl)formohydrazonate. The crude product is dissolved in 50 mL dichloromethane, treated with 1.2 eq SCl₂ and after extractive workup, crude *N*-(2,2,2-trifluoroacetyl)formohydrazonic hypochlorous thioanhydride is obtained.

### Example 7:2-(trifluoromethyl)-1,3,4-thiadiazole

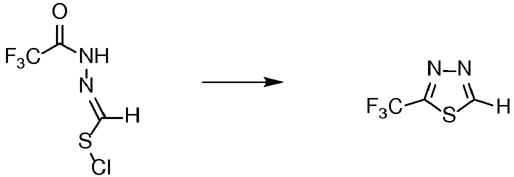

*N*-(2,2,2-trifluoroacetyl)formohydrazonic hypochlorous thioanhydride (1.0 eq) is treated with tBuOK (1.1 eq) in THF. After extractive workup, 2-(trifluoromethyl)-1,3,4-thiadiazole is obtained.

### Example 8 :Methyl 2-(2,2,2-trifluoroacetyl)hydrazine-1-carbodithioate

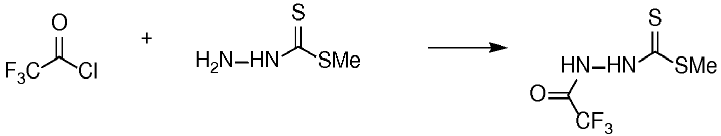

13.25 g (0.1 mol) trifluoroacetyl chloride are added to 12.2 g (0.1 mol) methyl hydrazinecarbodithioate in 50 mL dichloromethane at room temperature over a time of 2 hours. The obtained methyl 2-(2,2,2-trifluoroacetyl)hydrazine-1-carbodithioate is used in subsequent reactions without further purification; the content of the solution can be determined by NMR.

### Example 9 : 2-(methylthio)-5-(trifluoromethyl)-1,3,4-thiadiazole

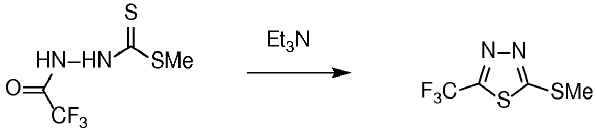

10.9 g (0.05 mol) of the methyl 2-(2,2,2-trifluoroacetyl)hydrazine-1-carbodithioate obtained in the example 8 in dichloromethane are treated with 30.4 g (0.3 mol) triethylamine. The obtained 2-(methylthio)-5-(trifluoromethyl)-1,3,4-thiadiazole is used in subsequent reactions without further purification; the content of the solution can be determined by NMR.

### Example 10: 2-(methylsulfonyl)-5-(trifluoromethyl)-1,3,4-thiadiazole

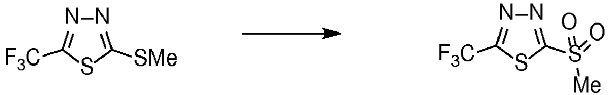

6 g (0.03 mol) of the 2-(methylthio)-5-(trifluoromethyl)-1,3,4-thiadiazole obtained in the example 9 in dichloromethane are treated with 4 g (0.12 mol) H₂O₂ (13.3. mL aq. 30% H₂O₂ solution) overnight at room temperature. The phases are separated, the organic phase is dried over Na₂SO₄, and the 2-(methylsulfonyl)-5-(trifluoromethyl)-1,3,4-thiadiazole is purified in a Kugelrohr distillation.

### Example 11 :N-(4-fluorophenyl)-N-isopropyl-2-((5-(trifluoromethyl)-1,3,4-thiadiazol-2-yl)oxy)acetamide

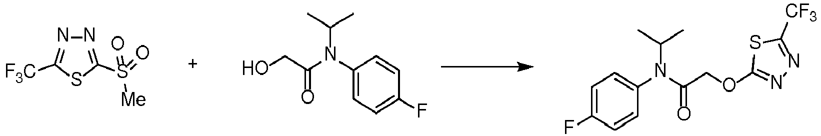

11.6 g (0.05 mol) 2-(methylsulfonyl)-5-(trifluoromethyl)-1,3,4-thiadiazole obtained in the example 10 is treated with 50 mL TDA/sulfolane, and N-(4-fluorophenyl)-2-hydroxy-N-isopropylacetamide are added. The mixture is heated to 65°C for 3 hours, cooled to room temperature and treated with ice water. After phase separation and drying of the organic phase, the volatiles are evaporated any the residue is recrystallized to obtain the N-(4-fluorophenyl)-N-isopropyl-2-((5-(trifluoromethyl)-1,3,4-thiadiazol-2-yl)oxy)acetamide.

## Claims

1. Process for the manufacture of a compound of formula (I), which comprises a step wherein a compound of formula (II) is reacted with a compound of formula (III) 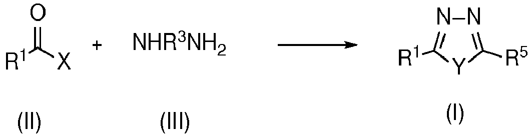
wherein R¹ is a halogenated C₁₋₄ alkyl group,
Y is -S- or -S(O₂)-, R³ is selected from the group consisting of H and - C(S)-R², wherein R² is the group -QR⁴ wherein R⁴ is a C₁₋₄ alkyl group,
wherein when R³ is -C(S)-R², wherein Q is S or O, and R⁴ is a C₁₋₄ alkyl group, R⁵ in (I) is the group R²
wherein when R³ is H, R⁵ in (I) is the group H
and wherein X is selected from the group consisting of F, Cl, Br and I.

2. Process for the manufacture of a compound of formula (XIV)), which comprises a step wherein a compound of formula (II) is reacted with a compound of formula (III) 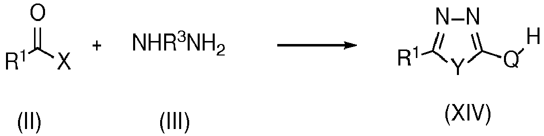 wherein R¹ is a halogenated C₁₋₄ alkyl group, Y is -S- or -S(O₂)-, wherein R³ is -C(S)-R², wherein R² is the group -QR⁴ wherein Q is S or O, and R⁴ is a C₁₋₄ alkyl group, and wherein a base is present in the reaction of (II) and (III).

3. Process according to claim 1 or 2, wherein R¹ is a fluorinated C₁₋₄ alkyl group.

4. Process according to claim 3, wherein R¹ is selected from the group consisting of CF₃, CF₂H, CF₂Cl, CFCl₂ and CCl₃, wherein CF₃ is preferred.

5. Process according to anyone of claims 1 to 4, wherein X is Cl.

6. Process according to anyone of claims 1, 3, 4 or 5, wherein R³ in (III) is H, the compound of formula (IV) obtained by the reaction of (II) and (III) is reacted with CH(OR⁶)₃, wherein R⁶ is an optionally substituted C₁₋₄ alkyl group, 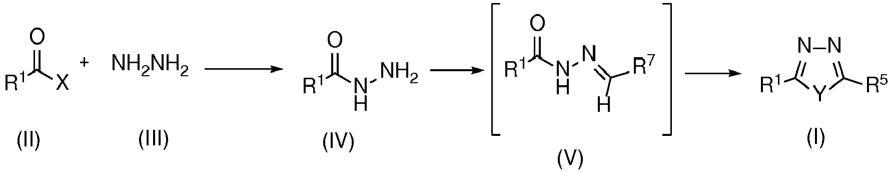 with a subsequent reaction with a compound selected from the group consisting of SCl₂, S₂Cl₂, SO₂Cl₂ or SO₂ClF to obtain the compound of formula (V), wherein R⁷ in the compound of formula (V) is -SCl or -S(O)₂Cl, which is cyclized, optionally in the presence of a base, into compound (I), wherein R⁵ is H and R¹ and Y are as defined in claim 1.

7. Process for the manufacture of a compound of formula (VI), 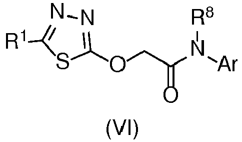 which comprises the process according to claim 6, wherein the compound of formula (IV) is reacted with CH(OR⁶)₃, with a subsequent reaction with a compound SCl₂ or S₂Cl₂ to obtain the compound of formula (V) wherein R⁷ is - SCl, which is cyclized, optionally in the presence of a base, into compound (I) wherein R⁵ is H, R¹ is as defined in claim 1 and Y is -S-, and which further comprises a step of halogenating the compound of formula (I) as obtained in the previous step with a halogenating agent 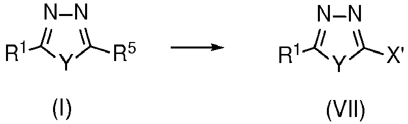 wherein X' is selected from F, Cl and Br
and which comprises a further step in which a compound of formula (VIII) is reacted with a compound of formula (VII), optionally in the presence of a base, to obtain a compound of formula (VI) 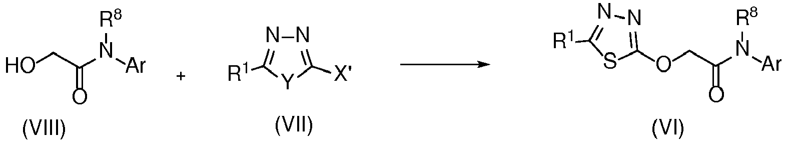 wherein
R⁸ is selected from the group consisting of H, optionally substituted branched or straight C₁-C₁₂-alkyl, optionally substituted C₃₋₈ cycloalkyl, optionally substituted aryl, optionally substituted heterocycloalkyl, and optionally substituted heteroaryl
wherein Ar is an optionally substituted aryl or heteroaryl group.

8. Process for the manufacture of a compound of formula (VI), 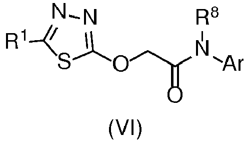 which comprises the process according to claim 6, wherein the compound of formula (IV) is reacted with CH(OR⁶)₃, with a subsequent reaction with a compound -SO₂Cl₂ or -SO₂ClF to obtain the compound of formula (V) wherein R⁷ is -SO₂Cl, which is cyclized, optionally in the presence of a base, into compound (I) wherein R⁵ is H, R¹ is as defined in claim 1 and Y is -S(O)₂-,
which further comprises a step wherein the compound of formula (I) wherein Y is -S(O)₂- and R⁵ is H is reduced with a reducing agent to a group of Y which is -S- 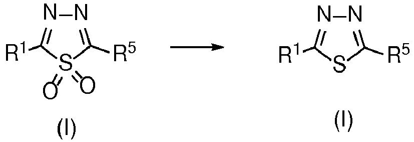 which further comprises a step of halogenating the compound of formula (I) with a halogenating agent 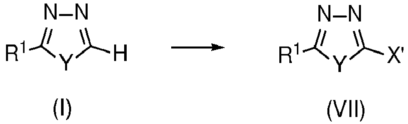 wherein X' is selected from F, Cl and Br
and which comprises a further step in which a compound of formula (VIII) is reacted with a compound of formula (VII), optionally in the presence of a base, to obtain a compound of formula (VI) 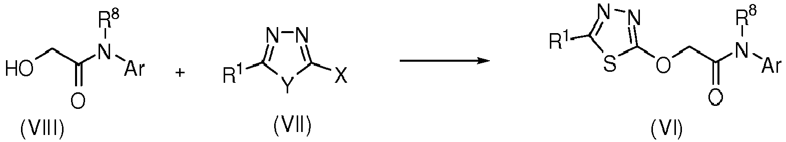 wherein
R⁸ is selected from the group consisting of H, optionally substituted branched or straight C₁-C₁₂-alkyl, optionally substituted C₃₋₈ cycloalkyl, optionally substituted aryl, optionally substituted heterocycloalkyl, and optionally substituted heteroaryl
R¹ is defined as in anyone of claims 1 to 5,
wherein Ar is an optionally substituted aryl or heteroaryl group.

9. Process according to anyone of claims 1, 3, 4, or 5, wherein, in the compound of formula (III), R³ is -C(S)-R², R² is the group -QR⁴, wherein Q is S or O, and R⁴ is a C₁₋₄ alkyl group, and wherein in the reaction with compound (II), a compound of formula (IX) is obtained, 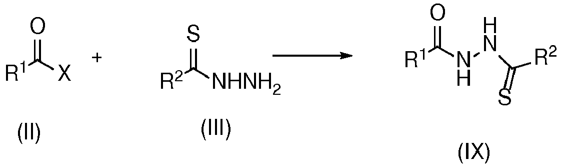 wherein the compound of formula (IX) then is cyclized, optionally in the presence of a base, to obtain a compound of formula (I) where Y is S and R¹ is a defined in any of the preceding claims 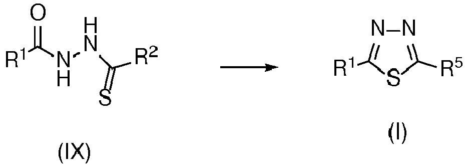 wherein R⁵ in compound (I) the group R², which is the group -QR⁴, wherein Q is S or O, and wherein R⁴ is a C₁₋₄ alkyl group.

10. Process for the manufacture of a compound of formula (VI), 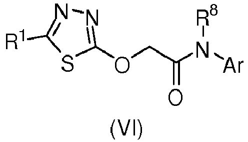 which comprises the process according to claim 9, wherein the compound of formula (I) in which R¹ is defined in any of the claims 1 to 9, and R² is the group -QR⁴ wherein Q is S, and wherein R⁴ is a C₁₋₄ alkyl group, is reacted with and oxidant to obtain a compound of formula (X) 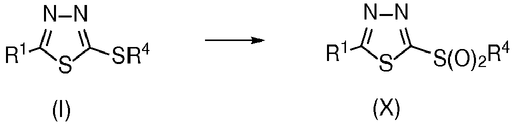 wherein R¹ is defined as above,
and wherein the compound of formula (X) is subsequently reacted with a mixture comprising a base and sulfone (X), followed by a reaction with a compound of formula (VIII) 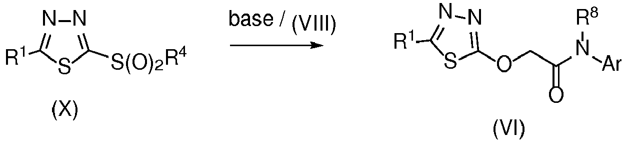 wherein the compound according to formula (VIII) is defined as in any of the preceding claims.

11. Process for the manufacture of a compound of formula (VI), 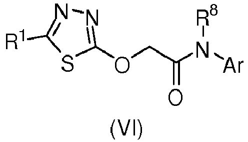 which comprises the process according to anyone of claims 2, 3, 4, 5 or 9,
which comprises a step wherein the compound of formula (I) in which R¹ is defined in any of the preceding claims, and R² is the group -QR⁴ wherein Q is S, and wherein R⁴ is a C₁₋₄ alkyl group,
or which comprises the step wherein a compound of formula (XIV) wherein Q is O,
is reacted with a compound of formula (XI), optionally in the presence of a base to obtain a compound of formula (XII) 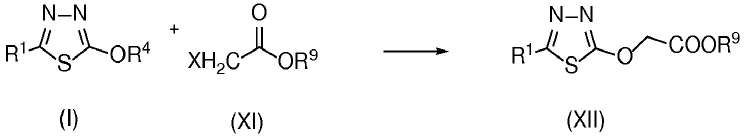 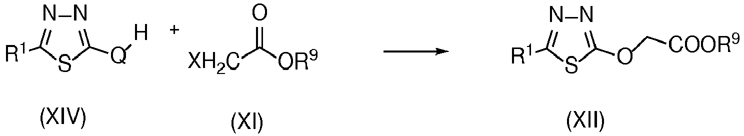 wherein X in compound (XI) is selected from F, Cl and Br, and wherein R⁹ is a C₁₋₁₂ alkyl group
and which further comprises a step of reacting the compound of formula (XII) with a compound of formula (XIII), optionally in the presence of a base, to obtain the compound of formula (VI)

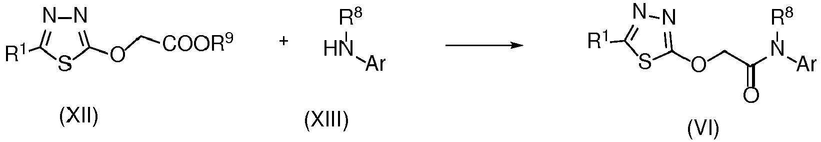

12. Process according to claim 11, wherein the compound of formula (XII) is converted into a compound of formula (XV) prior to the reaction with the compound of formula (XIII), with subsequent activation by reaction with a halogenating agent or activation as anhydride to obtain a compound of formula (XVI), before reaction with the compound of formula (XIII) to obtain the compound of formula (VI), wherein R¹⁰ is selected from the group consisting of X', wherein X' is selected from the group consisting of Cl, Br or F, and the group -O-C(O)-R¹¹, wherein R¹¹ is an optionally substituted C₁₋₄ alkyl group or an optionally substituted aryl group
or R¹⁰ is the group

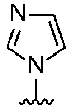

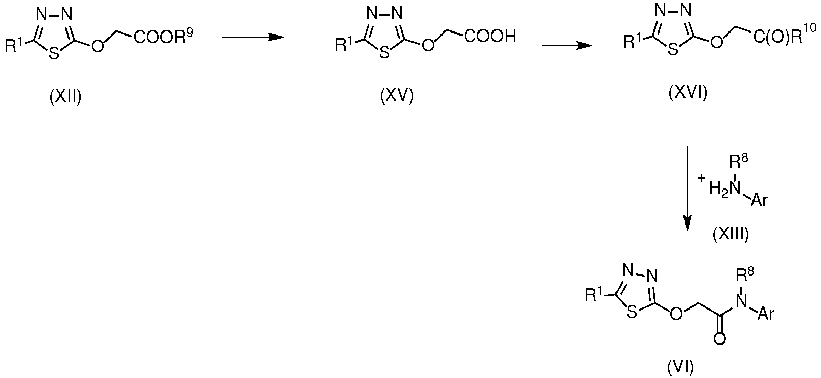

13. Process according to anyone of claims 7 to 12, wherein R⁸ is a C₁₋₄ alkyl group, preferably R⁸ is an isopropyl group.

14. Process for the manufacturing of an agriculturally or pharmaceutically active compound or a composition comprising an agriculturally or pharmaceutically active compound, which comprises at least one of the process according to anyone of claims 1 to 13.

15. Process according to claim 14, wherein the agriculturally active compound is N-(4-Fluorophenyl)-N-isopropyl-2-{ [5-(trifluoromethyl)-1,3,4-thiadiazol-2-yl]oxy}acetamide or a precursor thereof.
